# EUROPEAN PATENT APPLICATION

(11) **EP 3 884 845 A1**
(43) Date of publication of application: **29.09.2021**
(21) Application number: 21169153.0
(22) Date of filing: 17.03.2016
(51) Int. Cl.: A61B 3/10, A61B 3/103, A61B 3/00, A61B 3/107

(54) **OPHTHALMOLOGIC APPARATUS**

(30) Priority: 30.03.2015 JP 2015068981
(62) Divisional of application: 16160855.9
(71) Applicant: Kabushiki Kaisha Topcon, Tokyo 174-8580 (JP)
(72) Inventor: YOKO, Tatara, Tokyo, 174-8580 (JP); SHUNICHI, Morishima, Tokyo, 174-8580 (JP)
(74) Representative: Vossius & Partner Patentanwälte Rechtsanwälte mbB

(57) **Abstract**

The invention provides a method for testing a subject's eye with an optical apparatus having an optical system, the optical system comprising an ocular axial length measurement system (8), a topographic measurement unit (3), a refractometry measurement unit (6), and a processor, wherein a central axial length and a peripheral axial length of subject's eye is measured with the ocular axial length measurement system (8), wherein the topographic measurement unit (3) measures the shape of the cornea (K) of the eye, wherein the refractometry measurement unit (6) measures a refractive power of the subject's eye, wherein the processor (9) processes measurement data of the ocular axial length measurement system (8), the topographic measurement unit (3), and the refractometry measurement unit (6), wherein the processor (9) outputs the measurement data.

## Description

### FIELD

Embodiments described herein relate generally to an ophthalmologic apparatus.

### BACKGROUND

Cataract is a disease of an eye where the crystalline lens loses its transparency and becomes opaque or cloudy resulting in a gradual decrease of vision. For the eye with advanced cataract, generally, surgery is performed. For example, in the cataract surgery, the cloudy crystalline lens is removed, and is replaced with an intraocular lens (IOL) implant. There are several types of IOLs such as those with only a spherical degree, toric IOLs capable of correcting the astigmatism, multifocal IOLs capable of focusing on both distant and near objects, and the like. Before the cataract surgery, eye information, which represents the structure of the eye such as the axial length, is acquired by using an ophthalmologic apparatus. The power of IOL is determined based on the eye information.

For example, Japanese Patent No. 4523338 discloses such an ophthalmologic apparatus. Specifically, Japanese Patent No. 4523338 discloses an ophthalmologic apparatus that includes an optical system for measuring the ocular axial length and an optical system for measuring the refractive power of the eye, and thus can measure both the axial length and the refractive power of the eye.

Since the transmittance of the lens decreases due to turbidity in the eye with a cataract, the light irradiated onto the eye for measurement diffuses. Accordingly, a sufficient amount of light may not reach the fundus, or return light from the fundus cannot be detected sufficiently. As a result, in conventional ophthalmologic apparatuses, even if light is irradiated onto the eye with advanced cataract, the measurement may not be accomplished, and therefore the power of IOL may not be determined.

### SUMMARY

The object of the embodiments is to provide an ophthalmologic apparatus that can obtain the power of IOL even for the eye with advanced cataract.

According to one embodiment, an ophthalmologic apparatus includes a first measurement unit, a second measurement unit, and a controller. The first measurement unit is configured to irradiate a subject's eye with light from a first light source and detect return light thereof from the subject's eye to measure the refractive power of the subject's eye. The second measurement unit is configured to split light from a second light source into reference light and measurement light, irradiate the subject's eye with the measurement light, and detect interference light between return light of the measurement light from the subject's eye and the reference light to obtain at least the spherical diopter power of the subject's eye. The controller is configured to control the second measurement unit to perform a new measurement based on a measurement result obtained by the first measurement unit.

### BRIEF DESCRIPTION OF THE DRAWINGS

Fig. 1 is a schematic diagram illustrating an example of the configuration of an ophthalmologic apparatus according to an embodiment;
Fig. 2 is a schematic diagram illustrating an example of the configuration of an optical system of the ophthalmologic apparatus;
Fig. 3 is a schematic diagram illustrating an example of the configuration of the optical system of the ophthalmologic apparatus;
Fig. 4 is an explanatory diagram illustrating an example of the operation of the optical system of the ophthalmologic apparatus;
Fig. 5 is an explanatory diagram illustrating an example of the operation of the optical system of the ophthalmologic apparatus;
Fig. 6 is an explanatory diagram illustrating an example of the operation of the optical system of the ophthalmologic apparatus;
Fig. 7 is an explanatory diagram illustrating an example of the operation of the optical system of the ophthalmologic apparatus;
Fig. 8 is a schematic diagram illustrating an example of the configuration of a control system of the ophthalmologic apparatus;
Fig. 9 is a schematic diagram illustrating an example of the configuration of the control system of the ophthalmologic apparatus;
Fig. 10 is a flowchart of an example of the operation of the ophthalmologic apparatus of the embodiment;
Fig. 11 is a flowchart of an example of the operation of the ophthalmologic apparatus of the embodiment;
Fig. 12 is a flowchart of an example of the operation of the ophthalmologic apparatus of the embodiment;
Fig. 13 is a flowchart of an example of the operation of the ophthalmologic apparatus of the embodiment;
Fig. 14 is a flowchart of an example of the operation of an ophthalmologic apparatus according to a modification;
Fig. 15 is an explanatory diagram illustrating an example of the operation of the ophthalmologic apparatus of the modification; and
Fig. 16 is an explanatory diagram illustrating an example of the operation of the ophthalmologic apparatus of the modification.

### DETAILED DESCRIPTION

In the following embodiments, an ophthalmologic apparatus is described as being capable of objective and subjective measurements. In the objective measurement, information about a subject's eye is acquired using mainly a physical technique without reference to a response from the subject. The objective measurement includes measurement to obtain a value related to the subject's eye, and imaging to capture an image of the subject's eye. Examples of the objective measurement include objective refractometry (refractive power measurement), corneal shape measurement, tonometry, fundus photography, optical coherence tomography (OCT). In the subjective measurement, a result is obtained based on a response from the subject. Examples of the subjective measurement include subjective refractometry such as far vision test, near vision test, contrast test, and glare test, and visual field test. In the subjective measurement, information (targets, eye test charts, etc.) is presented to the subject, and a result is obtained based on a response of the subject to the information.

In this embodiment, an example is described in which using the technique of Fourier domain OCT is employed in OCT measurement. In particular, the ophthalmologic apparatus of the embodiment uses spectral domain OCT. Incidentally, the technique of OCT other than spectral domain OCT such as swept source OCT may be used. Further, the technique of time domain OCT may also be employed.

Besides, the ophthalmologic apparatus of the embodiment is capable of performing at least one of any subjective measurement and any objective measurement. In the following, the ophthalmologic apparatus of the embodiment is described as being capable of the far vision test, the near vision test, and the like as the subjective measurements, as well as the objective refractometry, corneal shape measurement, OCT measurement, and the like as the objective measurements. In the OCT measurement, eye information that represents the structure of the eye, such as the ocular axial length, the corneal thickness, the anterior chamber depth, and the lens thickness, is acquired. The following describes the case of obtaining the ocular axial length as the eye information in the OCT measurement. The configuration of the ophthalmologic apparatus of the embodiment is not limited to that described below. For example, an embodiment may be configured to perform the objective refractometry by means of another apparatus, and receive the measurement result from this apparatus and measure the ocular axial length by using the measurement result.

### <External structure>

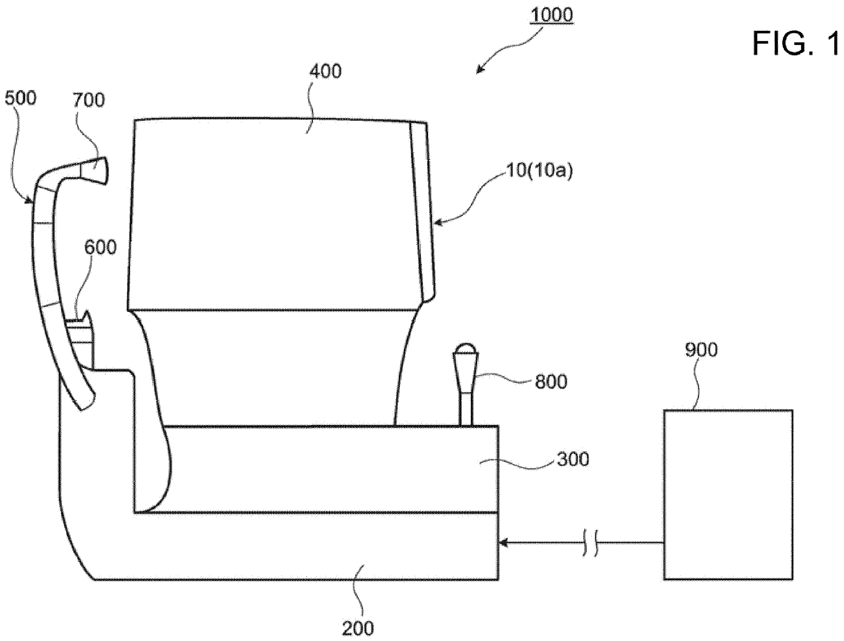

Fig. 1 illustrates an external structure of an ophthalmologic apparatus 1000 according to an embodiment. The ophthalmologic apparatus 1000 includes a base 200, a stand 300, a head portion 400, a face supporting portion 500, a joystick 800, and a display 10.

The stand 300 is movable in the anteroposterior and lateral directions with respect to the base 200. The head portion 400 is formed integrally with the stand 300. The face supporting portion 500 is formed integrally with the base 200.

The face supporting portion 500 is provided with a chin rest 600 and a forehead pad 700. The face supporting portion 500 makes the face of a subject (not illustrated) steady. For example, the examiner conducts a test at a position on the opposite side of the subject across the ophthalmologic apparatus 1000. The joystick 800 and the display 10 are located on the examiner's side. The joystick 800 is arranged on the stand 300. The display 10 is arranged on the surface of the head portion 400 on the examiner's side. The display 10 is, for example, a flat panel display such as a liquid crystal display. The display 10 has a display screen 10a of a touch panel.

The head portion 400 is moved back and forth as well as left and right by tilting the joystick 800. In addition, the head portion 400 is moved vertically by rotating the joystick 800 around its axis. These operations change the position of the head portion 400 with respect to the subject's face, which is held to the face supporting portion 500. Incidentally, the head portion 400 is moved left and right, for example, to switch an object to be tested by the ophthalmologic apparatus 1000 from the left eye to the right eye and vice versa.

An external device 900 is connected to the ophthalmologic apparatus 1000. The external device 900 may be any device, and also connected to the ophthalmologic apparatus 1000 by any connection link (communication link. etc.). The external device 900 includes, for example, an eyeglass lens measurement device configured to measure the optical properties of the lens. The eyeglass lens measurement device measures the power of an eyeglass lens worn by the subject, and feeds the measurement data to the ophthalmologic apparatus 1000. The external device 900 may be any other ophthalmologic apparatus. Further, the external device 900 may include a device (reader) having the function of reading information from a recording medium or a device (writer) having the function of writing information to a recording medium.

As another example of the external device 900 may be cited a computer used in a medical institution. Examples of such hospital computers include a hospital information system (HIS) server, a Digital Imaging and Communications in Medicine (DICOM) server, a doctor terminal, and the like. The external device 900 may include a computer that is used in the outside of the medical institution. Examples of such an out-of-hospital computer include a mobile terminal, a personal terminal, a server and a terminal of the manufacturer of the ophthalmologic apparatus 1000, a cloud server, and the like.

### <Configuration of the optical systems>

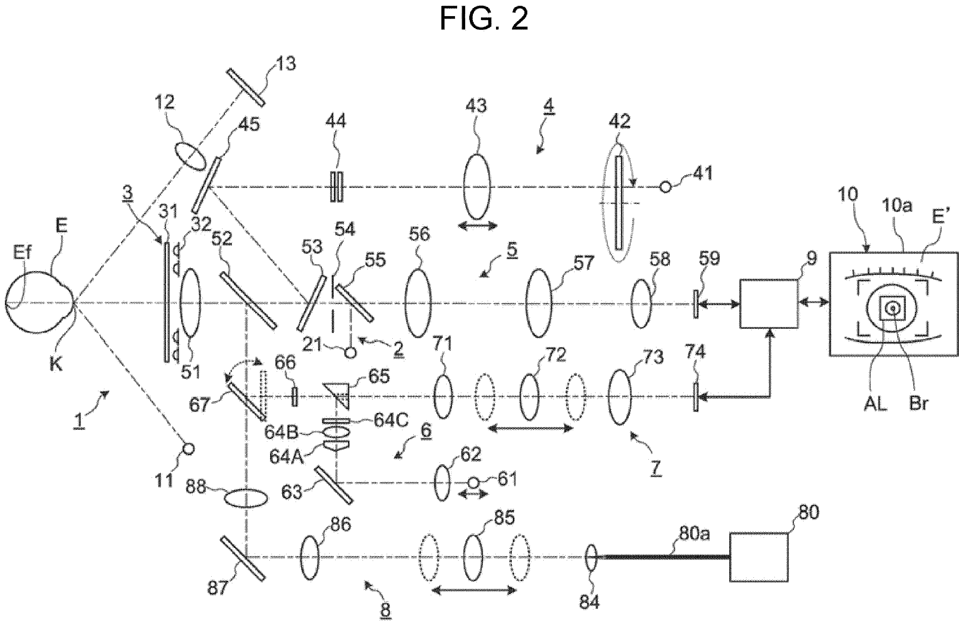

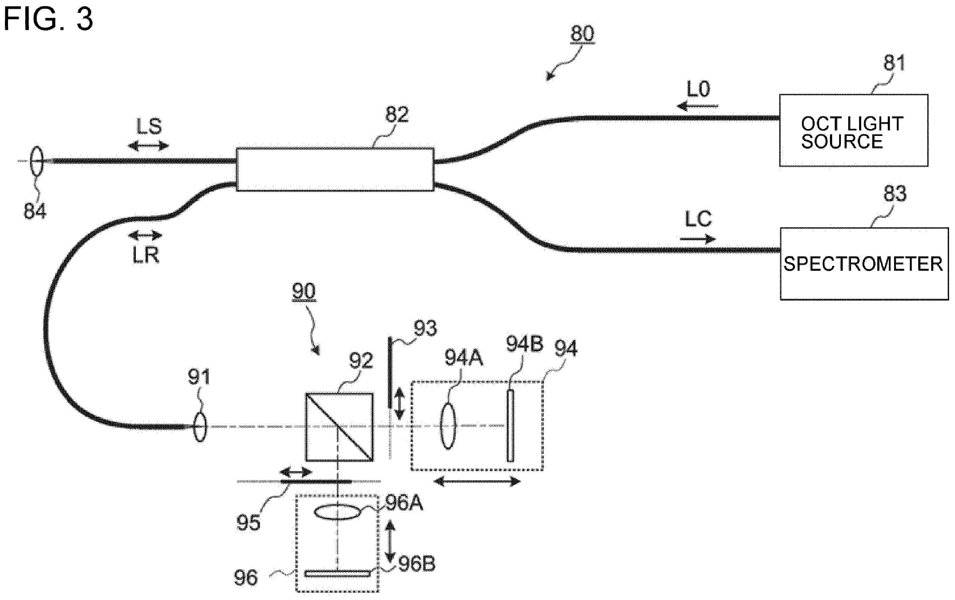

The ophthalmologic apparatus 1000 includes an optical system for testing the subject's eye. An example of the configuration of the optical system is described with reference to Figs. 2 to 7. The optical system is provided in the head portion 400. The optical system includes a Z-alignment projection system 1, an XY-alignment spot projection system 2, a keratometry ring projection system 3, a fixation and subjective measurement system 4, an observation system 5, a refractometry projection system 6, a refractometry light-receiving system 7, and an ocular axial length measurement system 8. A processor 9 performs a variety of processing.

The Z-alignment projection system 1 and the XY-alignment spot projection system 2 are optical systems for projecting the light necessary for the alignment of optical systems (XYZ-alignment) with respect to the subject's eye E. The Z-alignment projection system 1 has a function of performing alignment in a direction along the optical axis of the observation system 5 (front-back direction, Z direction). The XY-alignment spot projection system 2 has a function of projecting a spot for performing alignment in directions perpendicular to the optical axis of the observation system 5 (lateral direction, X direction; vertical direction, Y direction). The keratometry ring projection system 3 has a function of projecting, onto the subject's eye E, a ring-shaped light beam for measuring the shape of the cornea K of the subject's eye E. The fixation and subjective measurement system 4 is an optical system for presenting a fixation target and a visual target (optotype) for subjective measurement to the subject's eye E. The observation system 5 is an optical system for observing the anterior segment of the subject's eye E. The refractometry projection system 6 is an optical system for projecting a light beam for measuring the eye refractive power in an objective manner onto the subject's eye E. The refractometry light-receiving system 7 is an optical system for receiving the light that has been projected onto the subject's eye E by the refractometry projection system 6 and reflected by the fundus. The ocular axial length measurement system 8 is an optical system for measuring the ocular axial length of the subject's eye E by means of OCT measurement. The ocular axial length measurement system 8 has a function of projecting measurement light output from the OCT light source onto the fundus Ef, and a function of detecting the measurement light returning therefrom.

### (Observation system 5)

The observation system 5 includes an objective lens 51, dichroic mirrors 52 and 53, a diaphragm 54, a half mirror 55, relay lenses 56 and 57, an imaging lens 58, and an image sensor (CCD or the like) 59. The observation system 5 may further include an illumination light source for illuminating the anterior segment of the subject's eye E. The output from the image sensor 59 is fed to the processor 9. The processor 9 displays an anterior eye segment image E' on the display 10 based on the signal fed from the image sensor 59.

A keratometry board 31 is provided between the objective lens 51 and the subject's eye E. The keratometry board 31 is used to project a ring-shaped light beam for measuring the corneal shape onto the cornea K.

### (Z-alignment projection system 1 and XY-alignment spot projection system 2)

The Z-alignment projection system 1 is located in the periphery of the keratometry board 31. As described above, the Z-alignment projection system 1 is used for alignment in the front-back direction of the optical axis of the observation system 5. The Z-alignment projection system 1 includes a Z-alignment light source 11. The light emitted from the Z-alignment light source 11 is projected onto the cornea K. The light projected onto the cornea K is reflected by the cornea K, and is projected through an imaging lens 12 on a line sensor 13. When the position of the corneal apex changes in the front-back direction (that is, in the direction along the optical axis of the observation system 5), the position of a light beam projected on the line sensor 13 changes. By analyzing the change in the projection position, the position of the corneal apex of the subject's eye E relative to the objective lens 51 can be measured, and alignment can be performed based on the measured value.

The XY-alignment spot projection system 2 forms an optical path branched from the observation system 5 via the half mirror 55. As described above, the XY-alignment spot projection system 2 is used for alignment in the vertical and lateral directions. The XY-alignment spot projection system 2 includes an XY-alignment light source 21. The light output from the XY-alignment light source 21 is partly reflected by the half mirror 55 and passes through the diaphragm 54. Then, the light passes through the dichroic mirrors 53 and 52, and passes through the objective lens 51 to be projected onto the subject's eye E. The light projected onto the subject's eye E is reflected by the cornea K, and passes through the objective lens 51. Then the light travels through the optical path of the observation system 5, and is projected onto the image sensor 59.

As illustrated in Fig. 2 and the like, on the display screen 10a, an alignment mark AL and a bright spot image Br reflected by the cornea K are displayed together with the anterior eye segment image E'. When alignment is performed manually, the user operates the joystick 800 to adjust the position of the head portion 400 while referring to information displayed on the display screen 10a, for example. At this time, the processor 9 may calculate, for example, the amount of deviation in the vertical and lateral directions based on the amount of deviation between the alignment mark AL and the bright spot image Br reflected by the cornea K to display it on the display screen 10a. Further, the processor 9 may obtain the amount of deviation in the optical axis direction based on information from the Z-alignment projection system 1 to display it on the display screen 10a. The processor 9 can perform control to start measurement in response to the completion of the alignment.

When the alignment is performed automatically, a motorized mechanism is controlled to move the head portion 400 to thereby cancel the deviation. This mechanism includes an actuator for generating a driving force, and a member for transmitting the driving force to the head portion 400. The processor 9 can perform control to start measurement in response to the completion of the alignment.

### (Fixation and subjective measurement system 4)

The fixation and subjective measurement system 4 includes a light source 41, a transmissive target chart 42, a focusing lens 43, a variable cross cylinder (VCC) lens 44, and a reflective mirror 45. The focusing lens 43 can be moved along the optical axis of the fixation and subjective measurement system 4. The fixation and subjective measurement system 4 may further include the dichroic mirrors 53 and 52, and the objective lens 51.

The fixation and subjective measurement system 4 is able to project a target for visual acuity measurement onto the fundus Ef of the subject's eye E.

The light (visible light) output from the light source 41 is irradiated to the target chart 42. The target chart 42 represents a target for visual acuity measurement and a fixation target such as a landscape chart, and transmits the light output from the light source 41. The target chart 42 may include a transmissive liquid crystal panel. In this case, the fixation target and the target for visual acuity measurement are displayed on the liquid crystal panel. The light having transmitted through the target chart 42 transmits through the focusing lens 43 and the VCC lens 44, and is reflected by the reflective mirror 45 and the dichroic mirror 53. The light from the light source 41 reflected by the dichroic mirror 53 transmits through the dichroic mirror 52, passes through the objective lens 51, and is projected onto the fundus Ef of the subject's eye E. Thereby, the fixation target and the target for visual acuity measurement displayed on the target chart 42 are presented to the subject's eye E. In the objective refractometry, alignment is performed while the subject is staring at the landscape chart projected on the fundus Ef, and the eye refractive power is measured in a fogged vision state.

### (Refractometry projection system 6 and refractometry light-receiving system 7)

The refractometry projection system 6 and the refractometry light-receiving system 7 constitute a refractometry system. The refractometry projection system 6 has a function of projecting a ring-shaped measurement pattern light beam generated from the light (infrared light) output from a refractometry light source 61 onto the fundus Ef of the subject's eye E. The refractometry light-receiving system 7 has a function of receiving the measurement pattern light beam having been projected onto the subject's eye E by the refractometry projection system 6 and returning therefrom.

The refractometry projection system 6 includes the refractometry light source 61, a condenser lens 62, a reflective mirror 63, a conical prism 64A, a relay lens 64B, a ring aperture 64C, a perforated prism 65, a rotary prism 66, and a quick return mirror 67. The refractometry projection system 6 may further includes the dichroic mirror 52 and the objective lens 51. The refractometry light source 61 can be moved along the optical axis of the refractometry projection system 6. The refractometry light source 61 is located in a position substantially optically conjugate to the fundus Ef of the subject's eye E. Thereby, the light from the refractometry light source 61 passes through the cloudy crystalline lens, and easily reaches the fundus Ef.

The refractometry light-receiving system 7 includes a relay lens 71, a focusing lens 72, an imaging lens 73, and an image sensor (CCD or the like) 74. The refractometry light-receiving system 7 may further include the objective lens 51, the dichroic mirror 52, the quick return mirror 67, the rotary prism 66, and the perforated prism 65. The focusing lens 72 can be moved along the optical axis of the refractometry light-receiving system 7. The focusing lens 72, the refractometry light source 61, and a focusing lens 85 of the ocular axial length measurement system 8 (described later) are moved along their respective optical axis directions in an interlocking manner. The output from the image sensor 74 is fed to the processor 9. The objective lens 51, the dichroic mirror 52, the quick return mirror 67, the rotary prism 66, and the perforated prism 65 are shared with the refractometry projection system 6.

The light output from the refractometry light source 61 passes through the condenser lens 62, and is reflected by the reflective mirror 63. The light then transmits through the conical prism 64A and the relay lens 64B, and is guided to the ring aperture 64C. The light guided to the ring aperture 64C passes through a ring-shaped pattern portion to be a ring-shaped measurement pattern light beam. The conical prism 64A condenses the light from the refractometry light source 61, which has been condensed by the condenser lens 62, to the ring-shaped pattern portion of the ring aperture 64C. The measurement pattern light beam is reflected by the reflecting surface of the perforated prism 65, passes through the rotary prism 66, and is guided to the quick return mirror 67. With the use of the rotary prism 66, it becomes possible to uniformize the light amount distribution of the measurement pattern light beam to a blood vessel, a diseased site, or the like in the fundus Ef.

The quick return mirror 67 is used to switch between objective refractometry and ocular axial length measurement. At the time of the objective refractometry, the reflecting surface of the quick return mirror 67 is arranged on an optical path branched from the optical path of the observation system 5 by the dichroic mirror 52. Thereby, both the optical path of the refractometry projection system 6 and the optical path of the refractometry light-receiving system 7 are coupled to the optical path of the observation system 5. At the time of the ocular axial length measurement, the quick return mirror 67 is retracted from the optical path branched from the optical path of the observation system 5 by the dichroic mirror 52. Thereby, the optical path of the ocular axial length measurement system 8 is coupled to the optical path of the observation system 5.

Here, the reflecting surface of the quick return mirror 67 is arranged on an optical path branched from the optical path of the observation system 5 by the dichroic mirror 52. The measurement pattern light beam, which has been guided from the perforated prism 65 to the quick return mirror 67 through the rotary prism 66, is reflected by the quick return mirror 67. Subsequently, the measurement pattern light beam is reflected by the dichroic mirror 52, and passes through the objective lens 51 to be projected onto the subject's eye E.

The return light of the measurement pattern light beam projected onto the subject's eye E passes through the objective lens 51, and is reflected by the dichroic mirror 52 and the quick return mirror 67. Then, the return light passes through the rotary prism 66 and the center part of the perforated prism 65. Then, the return light transmits through the relay lens 71 and the focusing lens 72, and is formed into an image on the imaging surface of the image sensor 74 by the imaging lens 73.

### (Ocular axial length measurement system 8)

The ocular axial length measurement system 8 includes an OCT unit 80, a collimator lens 84, the focusing lens 85, a relay lens 86, an XY scanner 87, and a pupil lens 88. The ocular axial length measurement system 8 may further include the dichroic mirror 52 and the objective lens 51. The dichroic mirror 52 and the objective lens 51 are shared with the observation system 5.

The XY scanner 87 one-dimensionally or two-dimensionally deflects the light. By controlling the XY scanner 87, the incident position of measurement light LS (described later) can be changed with respect to the subject's eye E. To deflect the light in a two-dimensional manner, a first deflecting member for deflecting the light in a first direction and a second deflecting member for deflecting the light in a second direction may be used as the XY scanner 87. The first direction and the second direction are substantially perpendicular to each other. Examples of the deflecting member used as the XY scanner 87 include a galvanometer mirror, a polygon mirror, a rotation mirror, and the like. In addition, a dove prism, a double dove prism, a rotation prism, a micro-electro-mechanical systems (MEMS) mirror scanner, or the like may also be used. The XY scanner 87 is located in a position substantially optically conjugate to the fundus Ef of the subject's eye E.

The OCT unit 80 includes an OCT light source 81, a fiber coupler 82, and an optical path length changing unit 90. The OCT unit 80 may include a spectrometer 83. The focusing lens 85 can be moved along the optical axis of the ocular axial length measurement system 8.

The light (infrared light) output from the OCT light source 81 is split into reference light LR and the measurement light LS by the fiber coupler 82. The measurement light LS is output from the OCT unit 80, and is guided to the collimator lens 84 thorough an optical fiber. The reference light LR is guided to the optical path length changing unit 90 through an optical fiber. The optical path length changing unit 90 changes the optical path length of the reference light LR.

Here, the quick return mirror 67 is retracted from the optical path branched from the optical path of the observation system 5 by the dichroic mirror 52. The measurement light LS is collimated by the collimator lens 84 into a parallel light beam. Subsequently, the measurement light LS passes through the focusing lens 85 and the relay lens 86, and is deflected by the XY scanner 87. Then, the measurement light LS passes through the pupil lens 88, and is guided to the dichroic mirror 52. The measurement light LS guided to the dichroic mirror 52 is then reflected by the dichroic mirror 52, and transmits through the objective lens 51. Thereby, the measurement light LS is projected onto the fundus Ef of the subject's eye E.

The return light of the measurement light LS from the fundus Ef passes through the objective lens 51, and is guided to the OCT unit 80 via the same route as that of the measurement light LS toward the subject's eye E. The OCT unit 80 makes the return light of the measurement light LS and the reference light LR interfere with each other by means of the fiber coupler 82 to generate interference light LC. The optical path length changing unit 90 includes a collimator lens 91, a beam splitter 92, a retina shutter 93, a retinal reference mirror unit 94, a cornea shutter 95, and a corneal reference mirror unit 96. The retinal reference mirror unit 94 includes an imaging lens 94A and a reference mirror 94B. The corneal reference mirror unit 96 includes an imaging lens 96A and a reference mirror 96B. The beam splitter 92 splits the reference optical path, through which the reference light LR obtained by the fiber coupler 82 is guided, into a reference optical path for retina measurement and a reference optical path for cornea measurement. The interference light LC is guided into the spectrometer 83 through an optical fiber. In the spectrometer 83, the light that is spatially wavelength-separated is projected onto the line sensor. The processor 9 applies signal processing such as a known fast Fourier transform (FFT) to a signal output from the line sensor to thereby acquire information on the depth direction (Z direction).

Each part of the ophthalmologic apparatus 1000 is controlled by the processor 9. The processor 9 controls the Z-alignment light source 11, the XY-alignment light source 21, a keratometry ring light source 32 of the keratometry board 31, the light source 41, the refractometry light source 61, the OCT light source 81, the target chart 42, and the focusing lenses 43, 72, and 85. The processor 9 also controls the VCC lens 44, the quick return mirror 67, the optical path length changing unit 90, and the display 10, and the like.

### (Corneal shape measurement function)

When performing keratometry, the processor 9 turns on the keratometry ring light source 32. Reflection light of the ring-shaped light beam for measuring the corneal shape from the cornea K is projected onto the image sensor 59 by the observation system 5 together with an image of the anterior eye segment. The processor 9 applies predetermined arithmetic processing to the image captured by the image sensor 59 to calculate a parameter representing the shape of the cornea K.

### (Refractometry function)

When performing refractometry (objective refractive power measurement), the processor 9 turns on the refractometry light source 61. As described above, the light from the refractometry light source 61 is made into a ring-shaped measurement pattern light beam and projected onto the subject's eye E. If the subject's eye E is an emmetropia (= 0D (diopter)), the position where the refractometry light source 61 is conjugated to the fundus Ef of the subject's eye E is the reference position of the refractometry light source 61 and the focusing lens 72. The light beam projected onto the fundus Ef in this state is reflected by the fundus Ef, and a ring image based on the light returning from the fundus Ef is formed on the image sensor 74. Further, based on a measured value (refraction value) calculated from the ring image, the refractometry light source 61 and the focusing lens 72 are moved to a position where the refractometry light source 61 is substantially conjugate to the fundus Ef. The processor 9 analyzes the image based on the light returning from the fundus Ef detected by the image sensor 74. By adding the movement amount of the refractometry light source 61, the processor 9 calculates, as the refractive power of the subject's eye E, spherical diopter power S, astigmatic power C, and astigmatism axis angle A.

The refractometry light source 61 is, for example a super luminescent diode (SLD) that outputs broadband low-coherence light like. The refractometry light source 61 is, for example, a low coherence light source, the center wavelength of which is in a range of 820 nm to 880 nm. Thereby, the objective refractometry can be performed while the subject feels less burden of viewing the measurement light LS.

### (Axial length measurement function)

Ocular axial length measurement is performed using the OCT measurement function. When performing an ocular axial length measurement, the processor 9 measures the distance between the corneal apex of the subject's eye E and the retina as the ocular axial length while controlling the optical path length changing unit 90. First, the processor 9 turns on the OCT light source 81. The optical path length changing unit 90 is controlled in synchronization with the lighting of the OCT light source 81. More specifically, the cornea shutter 95 is inserted to the optical path between the beam splitter 92 and the corneal reference mirror unit 96. Meanwhile, the retina shutter 93 is retracted from the optical path between the beam splitter 92 and the retinal reference mirror unit 94. Light L0 from the OCT light source 81 is split into the reference light LR and the measurement light LS by the fiber coupler 82. As described above, the measurement light LS is projected onto the fundus Ef of the subject's eye E. At this time, the quick return mirror 67 has been retracted out of the optical path. Besides, from the result of the refractive power measurement of the subject's eye E as described above, the focusing lens 85 is moved such that an end face of a fiber 80a is conjugate to the fundus Ef of the subject's eye E. The light reflected by the fundus Ef returns the same optical path to be projected onto the end face of the fiber, and reaches the fiber coupler 82.

On the other hand, the reference light LR is collimated by the collimator lens 91 into parallel light, and split by the 50:50 beam splitter 92 into two light beams (for the cornea and the retina). The light beam for the retina obtained by the beam splitter 92, is converged on the reference mirror (reflective mirror) 94B by the imaging lens 94A. The light beam for the cornea obtained by the beam splitter 92 is converged on the reference mirror (reflective mirror) 96B by the imaging lens 96A. Here, as described above, the cornea shutter 95 has been inserted in the optical path, while the retina shutter 93 has been retracted from the optical path. Accordingly, only the light reflected from the reference mirror 94B returns the same optical path to reach the fiber coupler 82. The light reflected by the fundus Ef and that reflected by the reference mirror 94B are superposed by the fiber coupler 82, and guided to the spectrometer 83 as an interference signal (interference light LC). In the spectrometer 83, the light that is spatially wavelength-separated is projected onto the line sensor. The processor 9 applies signal processing such as a known FFT to a signal output from the line sensor to thereby acquire information on the depth direction.

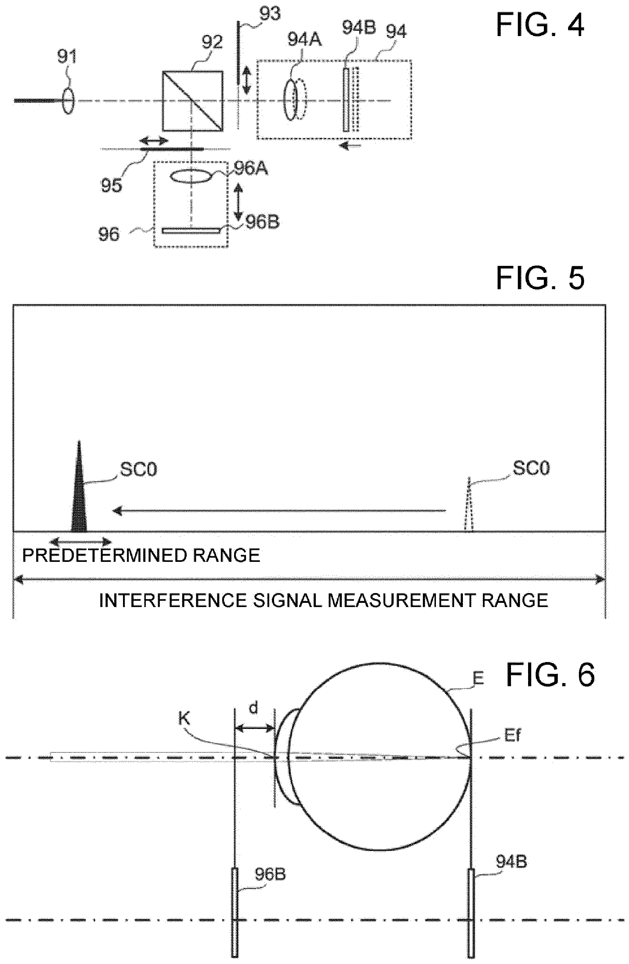

The retinal reference mirror unit 94 and the corneal reference mirror unit 96 are moved according to the ocular axial length of the subject's eye E such that the interference signal is located in a predetermined position in the depth direction. Fig. 5 illustrates an example of the intensity change of the interference signal obtained by the FFT with respect to the depth direction. In this case, by moving the retinal reference mirror unit 94 in the optical axis direction as illustrated in Fig. 4, the position of the interference signal SC0 corresponding to the retina can be moved to a predetermined position within a predetermined range as illustrated in Fig. 5. Here, the position of the reference mirror 96B may be fixed.

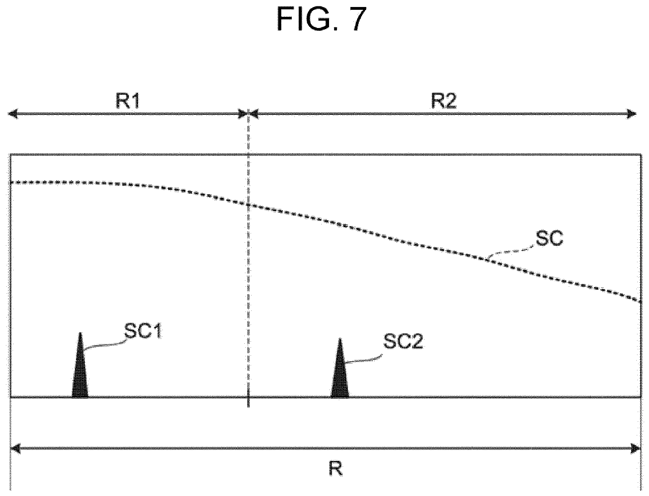

Further, since the coordinates of the corneal apex are detected by using the Z-alignment projection system 1, the distance between the corneal apex and the objective lens 51 (the working distance) can always be adjusted within a certain range. Here, when the cornea shutter 95 is retracted from the optical path an interference signal corresponding to the light reflected by the cornea K, of the light projected onto the subject's eye E, is simultaneously projected onto the spectrometer 83. If the working distance is within the predetermined range, the reference mirror 96B is located in a position at a distance d away from the corneal position so as not to superpose the interference signal SC1 corresponding to the retina (Fig. 6). Thus, as illustrated in Fig. 7 (illustrating the intensity change of the interference signal after the FFT with respect to the depth direction in the same manner as Fig. 5), two interference signals (the interference signal SC1 corresponding to the retina and the interference signal SC2 corresponding to the cornea) can be obtained at the same time within an interference signal measurement range R. In particular, if the signal sensitivity SC is changed as illustrated in Fig. 7, the interference signal SC1 corresponding to the retina with a relatively weak signal component is detected in a measurement range R1 of relatively high signal sensitivity, while the interference signal SC2 corresponding to the cornea with a relatively strong signal component is detected in a measurement range R2 of relatively low signal sensitivity. Thereby, the two interference signals can be simultaneously obtained with high accuracy.

Incidentally, if the OCT unit 80 has a configuration for swept-source OCT, a wavelength tunable light source is provided in place of the OCT light source 81 that outputs low coherence light, and there is provided no optical member for spectrally decomposing interference light. As to the configuration of the OCT unit 80, any known technique can be applied depending on the type of OCT.

The OCT light source 81 outputs broadband, low-coherence light L0 such as an SLD. For example, the OCT light source 81 is a low coherence light source, the center wavelength of which is in a range of 820 nm to 880 nm or 1040 nm to 1060 nm. Thereby, the OCT measurement can be performed while the subject feels less burden of viewing the measurement light.

Incidentally, a glass block and a density filter may be arranged in the optical path of the reference light LR between the collimator lens 91 and the reference mirrors 94B and 96B. The glass block and the density filter act as a delay unit for matching the optical path length (optical distance) of the reference light LR and that of the measurement light LS. In addition, the glass block and the density filter act as a means to match the dispersion characteristics of the reference light LR and the measurement light LS, and the dispersion characteristics of the retinal reference mirror unit 94 and the corneal reference mirror unit 96.

The spectrometer 83 includes, for example, a collimator lens, a diffraction grating, an imaging lens, and a CCD. The interference light LC having entered the spectrometer 83 is collimated into a parallel light beam by the collimator lens, and thereafter split into spectral components (spectrally decomposed) by the diffraction grating. The interference light LC thus split is projected on the imaging surface of the CCD through the imaging lens. Having received the interference light LC, the CCD converts it to an electrical detection signal, and outputs it to the processor 9. The processor 9 generates OCT information of the subject's eye E (e.g., image data, etc.) based on the detection signal from the CCD. This process includes noise removal (noise reduction), filtering, FFT, and the like as in conventional spectral domain OCT technologies.

If the OCT unit 80 has a configuration for swept-source OCT, instead of the spectrometer 83, for example, an optical brancher and a balanced photodiode (BPD). The interference light LC is split into a pair of interference light beams by the optical brancher. The BPD includes a pair of photodetectors. One of the photodetectors detects one of the interference light beams while the other of the photodetectors detects the other of the interference light beams. The difference between detection signals (detection results) obtained by the photodetectors is output to the processor 9.

Although a Michelson interferometer is employed in this embodiment, any type of interferometer such as a Mach-Zehnder interferometer may be used as appropriate.

### (Subjective measurement function)

When performing a subjective measurement, the processor 9 controls the target chart 42 to display a desired visual target (optotype). The processor 9 moves the focusing lens 43 to a position according to the result of objective measurement. Similarly, based on the astigmatic state (astigmatic power, astigmatism axis angle) of the subject's eye E obtained by the objective measurement, the processor 9 can control the VCC lens 44 to correct the astigmatic state. The astigmatic power can be changed by rotating two cylindrical lenses, which constitute the VCC lens 44, independently in opposite directions. The astigmatism axis angle can be changed by rotating the two cylindrical lenses in the same direction by the same angle.

When the examiner or the processor 9 selects an optotype or a visual target, the processor 9 controls the target chart 42 to display the desired visual target. The light from the target chart 42 travels through the focusing lens 43, the VCC lens 44, the reflective mirror 45, the dichroic mirrors 53 and 52, and the objective lens 51, and is projected onto the fundus Ef.

The subject provides a response to the visual target projected onto the fundus Ef. For example, in the case of a visual target for visual acuity test, the visual acuity of the subject's eye is determined according to the responses of the subject. For example, in the case of a visual target for astigmatism test, a dot chart is displayed, and the astigmatic power of the subject's eye is determined according to the responses of the subject. The selection of a visual target and subject's response to it are repeated at the discretion of the examiner or the processor 9. The examiner or the processor 9 determines the visual acuity or prescribed values (S, C, A) based on the responses from the subject.

The configuration of the fixation and subjective measurement system 4, the configurations of the Z-alignment projection system 1 and the XY-alignment spot projection system 2, the configuration of the keratometry system, the principle of eye refractive power measurement, the principle of subjective measurement, the principle of cornea shape measurement, and the like are well known. Thus, in this embodiment, detailed description thereof is not provided.

### (Configuration of information processing system)

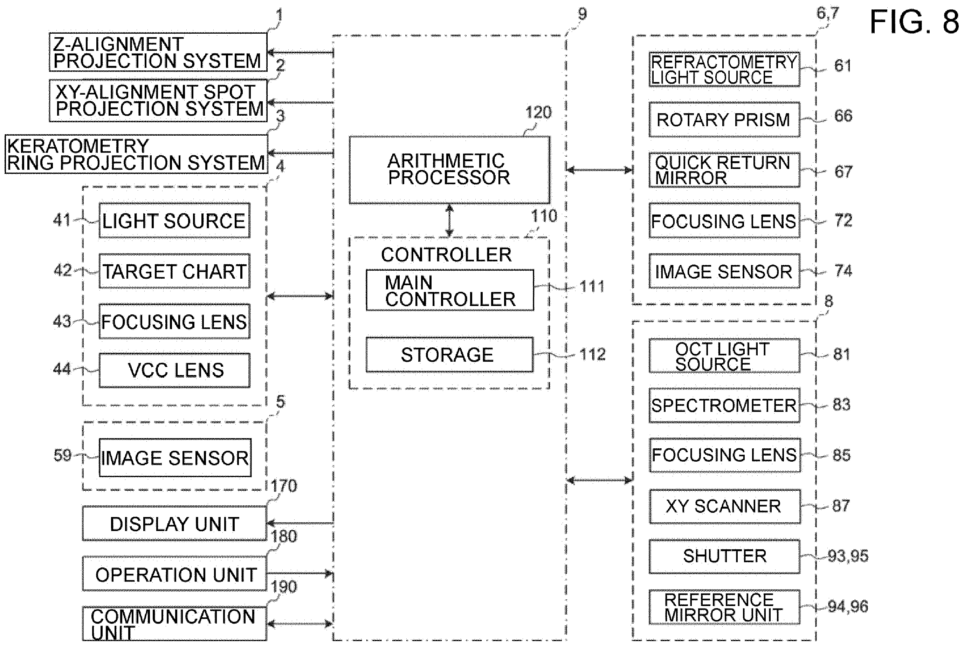

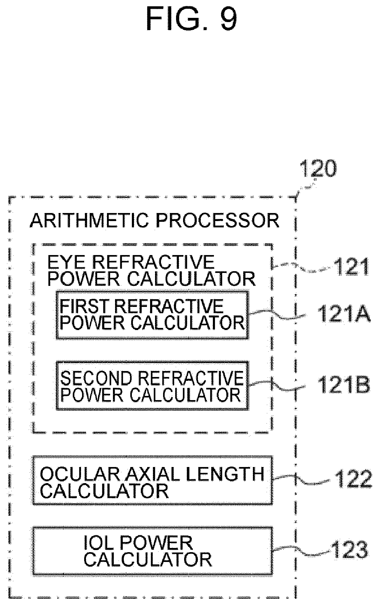

A description is given of an information processing system of the ophthalmologic apparatus 1000. Figs. 8 and 9 are functional block diagrams of an example of the configuration of the information processing system of the ophthalmologic apparatus 1000. The information processing system includes a controller 110, an arithmetic processor 120, a display unit 170, an operation unit 180, and a communication unit 190. The controller 110 controls the arithmetic processor 120, the Z-alignment projection system 1, the XY-alignment spot projection system 2, the keratometry ring projection system 3, the fixation and subjective measurement system 4, the observation system 5, the refractometry projection system 6, the refractometry light-receiving system 7, and the ocular axial length measurement system 8. Further, the controller 110 controls the display unit 170 and the communication unit 190. To control the Z-alignment projection system 1, the controller 110 controls the Z-alignment light source 11 and the line sensor 13. To control the XY-alignment spot projection system 2, the controller 110 controls the XY-alignment light source 21. To control the keratometry ring projection system 3, the controller 110 controls the keratometry ring light source 32. The processor 9 includes, for example, the controller 110 and the arithmetic processor 120.

### (Controller 110)

The controller 110 includes a main controller 111 and a storage 112. The controller 110 includes, for example, a microprocessor, a random access memory (RAM), a read only memory (ROM), a hard disk drive, and the like.

### (Main controller 111)

The main controller 111 performs various types of control for the ophthalmologic apparatus 1000. The main controller 111 controls the Z-alignment light source 11 and the line sensor 13 of the Z-alignment projection system 1, the XY-alignment light source 21 of the XY-alignment spot projection system 2, and the keratometry ring light source 32 of the keratometry ring projection system 3. This changes the amount of light output from the Z-alignment light source 11, the XY-alignment light source 21, and the keratometry ring light source 32, or switches the lighting/non-lighting state of them. Besides, having received a signal detected by the line sensor 13, the main controller 111 performs alignment control or the like based on the signal.

The main controller 111 controls the light source 41, the target chart 42, the focusing lens 43, and the VCC lens 44 of the fixation and subjective measurement system 4. This changes the amount of light output from the light source 41, switches the lighting/non-lighting state thereof, displays a visual target, a fixation target, and the like on the target chart 42, or changes the position of the focusing lens 43 in the optical axis direction. In addition, the astigmatism state of the subject's eye E is corrected by the VCC lens 44.

The main controller 111 controls the image sensor 59 of the observation system 5 to acquire a signal captured by the image sensor 59. The arithmetic processor 120 forms an image based on the signal. Incidentally, when the observation system 5 includes the illumination light source, the main controller 111 can control it.

The main controller 111 controls the refractometry light source 61, the rotary prism 66, and the quick return mirror 67 of the refractometry projection system 6. This moves the refractometry light source 61 along the optical axis of the refractometry projection system 6, changes the amount of light output from the refractometry light source 61, switches the lighting/non-lighting state thereof, or the like. This also rotates the rotary prism 66, or switchs the optical path by the quick return mirror 67.

The main controller 111 controls the focusing lens 72 and the image sensor 74 of the refractometry light-receiving system 7. With this, the position of the focusing lens 72 is changed in the optical axis direction. In addition, the main controller 111 acquires a signal captured by the image sensor 74, and controls the arithmetic processor 120 to form an image based on the signal.

The main controller 111 can change at least one of the amount of light output from the refractometry light source 61, the exposure time and the detection sensitivity of the image sensor 74, and the number of images, to be synthesized, based on light detected by the image sensor 74. Thereby, conditions are changed for measurement using the refractometry projection system 6 and the refractometry light-receiving system 7.

The main controller 111 controls the OCT light source 81, the spectrometer 83, the focusing lens 85, the XY scanner 87, the retina shutter 93, the cornea shutter 95, the retinal reference mirror unit 94, and the corneal reference mirror unit 96 of the ocular axial length measurement system 8. This changes the amount of light output from the OCT light source 81, switches the lighting/non-lighting state thereof, changes the position of the focusing lens 85 in the optical axis direction, or the like. In addition, the main controller 111 acquires a signal captured by the spectrometer 83, and controls the arithmetic processor 120 to form an image based on the signal. This also changes the position of the focusing lens 85. Further, this also changes the incident position of the measurement light LS with respect to the subject's eye E, or changes the position of the reference mirror. The main controller 111 may move the focusing lens 85 along the optical axis of the ocular axial length measurement system 8 in conjunction with the refractometry light source 61 and the focusing lens 72. Further, the main controller 111 may move the focusing lens 43 along the optical axis in conjunction with the focusing lens 85.

The main controller 111 can change at least one of the amount of light output from the OCT light source 81, the exposure time and the detection sensitivity of the spectrometer 83, and the number of images, to be synthesized, based on light detected by the spectrometer 83. Thereby, conditions are changed for measurement using the ocular axial length measurement system 8.

The main controller 111 performs the process of writing data to and reading data from the storage 112.

### (Storage 112)

The storage 112 stores various types of data. Examples of the data stored in the storage 112 include image data of the OCT information, image data of a fundus image, eye information, and the like. The eye information contains information about a subject such as a patient ID and name, and information about the subject's eye such as identification information for identifying the left or right eye. When the eye refractive power of the subject's eye E is measured by using the ophthalmologic apparatus 1000 or another apparatus, measurement data is stored in the storage 112. The storage 112 also stores various programs and data for operating the ophthalmologic apparatus 1000.

### (Display unit 170, operation unit 180)

The display unit 170 displays information under the control of the controller 110. The display unit 170 includes the display 10 illustrated in Fig. 1 and the like.

The operation unit 180 is used for operating the ophthalmologic apparatus 1000. The operation unit 180 includes various hardware keys (the joystick 800, buttons, switches, etc.) provided to the ophthalmologic apparatus 1000. The operation unit 180 further includes various software keys (buttons, icons, menus, etc.) displayed on the touch-panel display screen 10a.

At least part of the display unit 170 and the operation unit 180 may be configured integrally. The touch-panel display screen 10a is a typical example of this.

### (Communication unit 190)

The communication unit 190 has a function for communicating with the external device 900 illustrated in Fig. 9. The communication unit 190 may be provided to, for example, the processor 9. The communication unit 190 has a configuration corresponding to the mode of communication with the external device 900.

### (Arithmetic processor 120)

As illustrated in Fig. 9, the arithmetic processor 120 includes an eye refractive power calculator 121, an ocular axial length calculator 122, and an IOL power calculator 123. The eye refractive power calculator 121 includes a first refractive power calculator 121A and a second refractive power calculator 121B. Further, the arithmetic processor 120 receives signals acquired by the line sensor 13, the image sensors 59 and 74, and the spectrometer 83, and performs various kinds of control, image formation, image analyse, or the like.

The eye refractive power calculator 121 calculates the refractive power of the subject's eye E. The eye refractive power calculator 121 obtains the refractive power of the subject's eye E in a refractive power measurement using the refractometry projection system 6 and the refractometry light-receiving system 7 as well as in a refractive power measurement using the ocular axial length measurement system 8. The main controller 111 can measure the refractive power of the subject's eye E with the ocular axial length measurement system 8 based on the result of the refractive power measurement using the refractometry projection system 6 and the refractometry light-receiving system 7. When the refractive power measurement is performed with the refractometry projection system 6 and the refractometry light-receiving system 7, the first refractive power calculator 121A calculates the refractive power of the subject's eye E. When the refractive power measurement is performed with the ocular axial length measurement system 8, the second refractive power calculator 121B calculates the refractive power of the subject's eye E.

The first refractive power calculator 121A analyzes the shape of a ring image based on return light, returning from the fundus, of a ring-shaped measurement pattern light beam having been projected onto the subject's eye E to thereby calculate the refractive power of the ocular optical system of the subject's eye E. The first refractive power calculator 121A analyzes, for example, an image in which a ring image based on the return light is depicted, and obtains the barycentric position of the ring image. The first refractive power calculator 121A can obtain the barycentric position of the ring image by calculating the distribution of the pixel values (brightness values) in the image. Next, the first refractive power calculator 121A checks the pixel values (brightness values) in a plurality of scanning directions extending from the barycentric position toward the outer circumference of the ring image to obtain the brightness distribution in each of the scanning directions. The first refractive power calculator 121A specifies the center position in a predetermined range of brightness values from the brightness distribution in each of the scanning directions to specify the ring image. The first refractive power calculator 121A then performs elliptic approximation processing on the specified ring image to specify an approximate ellipse. With a known equation using the lengths of the major axis and the minor axis of the approximate ellipse, the first refractive power calculator 121A obtains the spherical diopter power S, the astigmatic power C, and the astigmatism axis angle A. The first refractive power calculator 121A can obtain deformation or displacement of the ring image captured by the image sensor 74 with respect to a reference pattern to obtain the refractive power of the subject's eye E from the deformation or the displacement.

The second refractive power calculator 121B calculates at least the spherical diopter power of the subject's eye E by OCT measurement performed by using the ocular axial length measurement system 8. In this embodiment, the second refractive power calculator 121B can obtain spherical equivalent power as the spherical diopter power. The second refractive power calculator 121B finds a peak value of the detection signal of the interference light LC by moving a reference mirror (e.g., the reference mirror 94B) in OCT measurement, thereby specifying a peak position. The second refractive power calculator 121B calculates the spherical equivalent power based on the movement amount of the reference mirror from a reference position of 0D, and the deviation amount of the peak position from the reference position.

Further, the eye refractive power calculator 121 can analyze a keratometry ring image captured by the image sensor 59 of the observation system 5 to calculate the corneal refractive power, the corneal astigmatic power, and the corneal astigmatism axis angle. For example, the eye refractive power calculator 121 performs arithmetic processing on the keratometry ring image to calculate the corneal curvature radii of the strong principal meridian and the weak principal meridian of the anterior corneal surface. The eye refractive power calculator 121 calculates the corneal refractive power, the corneal astigmatic power, and the corneal astigmatism axis angle from the corneal curvature radii.

The ocular axial length calculator 122 calculates the ocular axial length of the subject's eye E by using the two interference signals (see Fig. 7) obtained with the ocular axial length measurement system 8.

The IOL power calculator 123 obtains the power of IOL using a known equation with the refractive power and the axial length of the subject's eye E. Incidentally, the IOL power calculator 123 may obtain the power of IOL by using eye information other than the eye refractive power and/or the ocular axial length. Examples of such eye information include the corneal thickness, the anterior chamber depth, the lens thickness, and the like.

The refractometry projection system 6, the refractometry light-receiving system 7, and the arithmetic processor 120, (the first refractive power calculator 121A) are an example of the "first measurement unit" of this embodiment. The refractometry projection system 6 and the refractometry light-receiving system 7 are an example of the "measurement optical system" of this embodiment. The image sensor 74 is an example of the "first detector" of this embodiment. The ocular axial length measurement system 8 and the arithmetic processor 120 (the second refractive power calculator 121B) are an example of the "second measurement unit" of this embodiment. The OCT unit 80 is an example of the "interference optical system" of this embodiment. The spectrometer 83 is an example of the "second detector" of this embodiment.

### <Operation example>

A description is given of an example of the operation of the ophthalmologic apparatus 1000 of the embodiment. In this embodiment, when the IOL power cannot be determined based on a measurement result obtained by the objective refractometry of the subject's eye E, at least the spherical diopter power of the subject's eye E is obtained by OCT measurement performed by using the ocular axial length measurement system 8. In the following, as the case where the IOL power cannot be determined, an example is described in which it is determined to be impossible to capture a ring image based on the return light of a ring-shaped measurement pattern light beam projected onto the subject's eye E. However, the embodiment is not limited thereto.

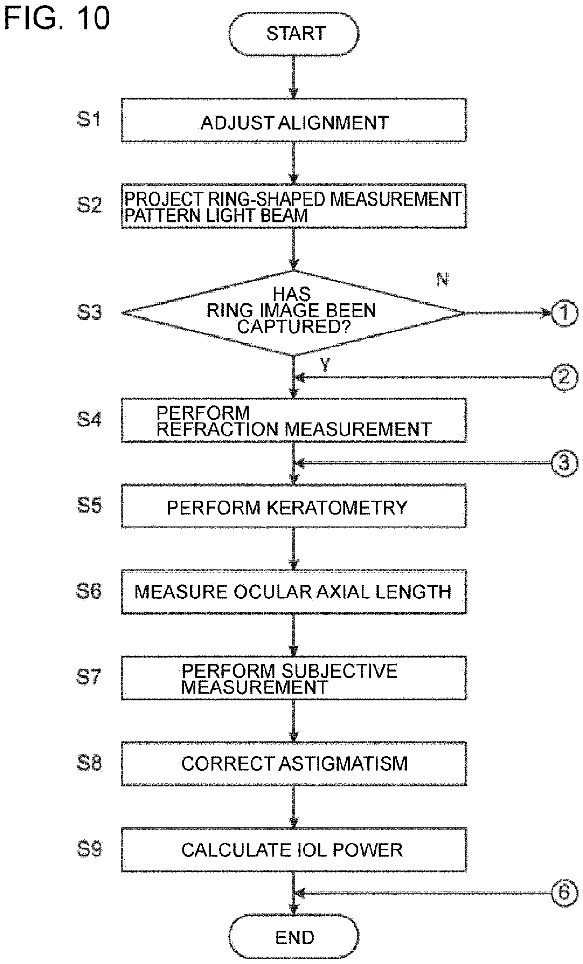

Figs 10 to 12 are flowcharts illustrating an example of the operation of the ophthalmologic apparatus 1000 of the embodiment.

### (S1)

First, after making the face of the subject stable by means of the face supporting portion 500, in response to the operation of the examiner performed on the operation unit 180, the controller 110 turns on the Z-alignment light source 11, the XY-alignment light source 21, and the light source 41. The processor 9 acquires an image signal of an image of the anterior eye segment formed on the imaging surface of the image sensor 59, and displays the anterior eye segment image E' on the display unit 170 (the display screen 10a of the display 10). Thereafter, the head portion 400 is moved to the test position of the subject's eye E. The test position refers to a position where the test of the subject's eye E can be carried out. The subject's eye E is positioned at the test position through the above-mentioned alignment (alignment by the Z-alignment projection system 1, the XY-alignment spot projection system 2, and the observation system 5). According to user's operation or an instruction from the user, or an instruction from the controller 110, the head portion 400 is moved under the control of the controller 110. That is, the head portion 400 is moved to the test position of the subject's eye E, and a preparation is made for carrying out an objective measurement.

Besides, under the control of the controller 110, the refractometry light source 61, the focusing lens 72, the focusing lens 85 and the focusing lens 43 are moved along the respective optical axes to the respective origins that is the respective positions corresponding to 0D, for example. The movements to the origins are made under the control of the controller 110 according to user's operation or an instruction from the user, or an instruction from the controller 110. When the user's operation or the instruction for focus adjustment is completed, or when the controller 110 determines that the focus adjustment is proper by a known method, the process moves to step S2.

### (S2)

Under the control of the controller 110, the reflecting surface of the quick return mirror 67 is arranged on an optical path branched from the optical path of the observation system 5 by the dichroic mirror 52. In step S2, a ring-shaped measurement pattern light beam for refractometry is projected onto the subject's eye E as described above. Thereby, based on the return light of the measurement pattern light beam from the subject's eye E, a ring image is formed on the imaging surface of the image sensor 74.

### (S3)

The controller 110 determines whether a ring image has been captured based on the return light from the fundus Ef detected by the image sensor 74 in step S2. For example, the controller 110 detects the positions (pixels) of the edge of the image based on the return light detected by the image sensor 74, and determines whether the width of the image (difference between the outer diameter and the inner diameter) is equal to or greater than a predetermined value. Alternatively, the controller 110 determines whether a ring can be formed based on points (image) with the intensity is equal to or greater than a predetermined value to determine whether a ring image has been captured. When it is determined that a ring image has been captured (Y in step S3), the process moves to step S4. On the other hand, if a ring image cannot be captured in the origin (for example, in the position corresponding to 0D), the refractometry light source 61, the focusing lens 72, and the focusing lenses 85 and 43 are moved in conjunction with one another to, for example, the positions corresponding to +10D, -10D, and the same determination is made. When it is determined that a ring image cannot be captured at any position (N in step S3), the process proceeds to step S10.

### (S4)

Having determined that a ring image can be captured (Y in step S3), the controller 110 performs refractometry. In the refractometry, the first refractive power calculator 121A analyzes, in the abovementioned manner, the ring image based on the return light of the measurement pattern light beam projected in step S2 or newly projected in step S4. Thereby, the first refractive power calculator 121A obtains the spherical diopter power S, the astigmatic power C, and the astigmatism axis angle A. The spherical diopter power and the like thus obtained are stored in the storage 112 of the controller 110. According to user's operation or instruction provided on the operation unit 180, or an instruction from the controller 110, the process proceeds to step S5.

### (S5)

The controller 110 turns on the keratometry ring light source 32. When light is output from the keratometry ring light source 32, a ring-shaped light beam for corneal shape measurement is projected onto the cornea K. The eye refractive power calculator 121 performs arithmetic processing on an image captured by the image sensor 59 to calculate the corneal curvature radii. From the corneal curvature radii thus calculated, the eye refractive power calculator 121 calculates the corneal refractive power, the corneal astigmatic power, and the corneal astigmatism axis angle. The corneal refractive power and the like thus obtained are stored in the storage 112 of the controller 110. According to user's operation or instruction provided on the operation unit 180, or an instruction from the controller 110, the process proceeds to step S6.

### (S6)

The controller 110 retracts the quick return mirror 67 from the optical path branched from the optical path of the observation system 5 by the dichroic mirror 52. Subsequently, the controller 110 turns on the OCT light source 81 to measure the ocular axial length in the abovementioned manner. The ocular axial length calculator 122 calculates the ocular axial length. The eye information thus obtained is stored in the storage 112 of the controller 110. Upon completion of the ocular axial length measurement, the process moves to step S7 according to user's operation or instruction provided on the operation unit 180, or an instruction from the controller 110.

### (S7)

For example, based on user's instruction provided on the operation unit 180, the controller 110 controls the target chart 42 to display a desired visual target. In addition, the controller 110 moves the focusing lens 43 to a position according to the result of the objective measurement. The subject provides a response to the visual target projected onto the fundus Ef. For example, in the case of a visual target for visual acuity test, the visual acuity of the subject's eye is determined by the response of the subject. The selection of a visual target and subject's response to it are repeated at the discretion of the examiner or the controller 110. Upon completion of the subjective measurement, the process proceeds to step S8 according to user's operation or instruction provided on the operation unit 180, or an instruction from the controller 110.

### (S8)

The controller 110 controls the VCC lens 44 based on the astigmatic state (astigmatic power, astigmatism axis angle) of the subject's eye E obtained by the objective measurement such that the astigmatism is to be corrected. The subject provides a response to the visual target projected onto the fundus Ef. For example, in the case of a visual target for visual acuity test, the visual acuity of the subject's eye is determined by the response of the subject. The selection of a visual target or the like and subject's response to it are repeated at the discretion of the examiner or the controller 110. The examiner or the controller 110 determines the visual acuity or prescribed values (S, C, A) based on the responses from the subject. When the correction is completed, the process proceeds to step S9 according to user's operation or instruction provided on the operation unit 180, or an instruction from the controller 110.

### (S9)

The controller 110 controls the IOL power calculator 123 to obtain the power of IOL by using the refractive power of the subject's eye E obtained by the above measurement and the ocular axial length obtained in step S6. The IOL power thus obtained is stored in the storage 112 of the controller 110. With this, the operation ends (END).

### (S10)

Having determined that a ring image cannot be captured in step S3 (N in step S3), the controller 110 retracts the quick return mirror 67 from the optical path branched from the optical path of the observation system 5 by the dichroic mirror 52. Subsequently, the controller 110 turns on the OCT light source 81. At this time, the focusing lens 85 is located in the position corresponding to 0D, for example.

### (S11)

The controller 110 searches for a peak value, which is equal to or higher than a predetermined crest value, of the detection signal of the interference light LC generated by the measurement light LS returning from the fundus Ef of the subject's eye E, while moving the reference mirror 94B of the retinal reference mirror unit 94 along the optical axis.

### (S12)

When it is determined that a peak value is detected by the search of the peak value (Y in step S12), the process proceeds to step S13. When the peak value is not detected in the origin (for example, in the position corresponding to 0D), the refractometry light source 61 and the focusing lens 72, and the focusing lenses 85 and 43 are moved in conjunction with one another to, for example, the position corresponding to +10D, -10D, and the same determination is made. When it is determined that no peak value is found at any position by the search of the peak value (N in step S12), the process proceeds to step S19.

### (S13)

Having determined that a peak value is detected in step S12 (Y in step S12), the controller 110 moves the focusing lens 85 such that a peak value of a higher crest value is to be detected. In conjunction with the focusing lens 85, the focusing lens 72 and the refractometry light source 61 are also moved along the optical axis of the refractometry light-receiving system 7.

### (S14)

The controller 110 calculates an evaluation value indicating the degree of progress of cataract from the new peak value obtained by the movement of the focusing lens 85 and the like in step S13.

For example, the degree of progress of cataract can be divided in advance into a plurality of stages according to the turbid state of the lens. An evaluation value is assigned to each stage in advance. The turbid state of the lens is assumed to be reflected to the peak value obtained in step S13. Therefore, the controller 110 may specify one of the stages corresponding to the peak value obtained in step S13 to determine the evaluation value indicating the degree of progress of cataract.

### (S15)

Next, the controller 110 changes conditions of the refractometry. For example, as a first example of change control in step S15, the controller 110 controls the image sensor 74 to increase the detection sensitivity by a first change amount. The first change amount may be a predetermined amount, or may be determined based on the evaluation value obtained in step S14. The increase in the detection sensitivity facilitates the detection of the return light from the fundus Ef of the subject's eye E. Thus, a ring image can be captured more easily.

In addition, as a second example of change control in step S15, the controller 110 may control the image sensor 74 to increase the exposure time by first change time, or to increase the number of images, to be synthesized, based on light detected by the image sensor 74 by a first change number. For example, the first change time and the first change number may be predetermined time and number, or may be determined based on the evaluation value obtained in step S14. The increase in the exposure time or in the number of images to be synthesized facilitates the detection of the return light from the fundus Ef of the subject's eye E. Thus, a ring image can be captured more easily.

Further, as a third example of change control in step S15, the controller 110 may control the refractometry light source 61 to increase the light amount by a second change amount. For example, the second change amount may be a predetermined amount, or may be determined based on the evaluation value obtained in step S14. The increase in the amount of light emitted from the light source increases the amount of light that reaches the fundus Ef of the subject's eye E. Thus, the amount of the return light can be increased.

In step S15, at least one of the first to third examples of change control is performed. At least one of the first to third examples of change control to be performed may be selected based on the evaluation value obtained in step S14. For example, one of the first to third examples of change control may be selected based on the evaluation value. Alternatively, priority order may be set for the first to third examples of change control in advance, and the control to be performed in step S15 may be determined according to the priority order.

### (S16)

Next, in the same manner as in step S3, the controller 110 determines whether a ring image has been captured based on the return light from the fundus Ef by refractometry, the conditions of which have been changed in step S15. When it is determined that a ring image has been captured (Y in step S16), the process moves to step S4, and the refractometry is performed again. On the other hand, when it is determined that a ring image cannot be captured (N in step S16), the process moves to step S17.

### (S17)

Having determined that a ring image cannot be captured (N in step S16), the controller 110 determines whether to perform a retry. For example, the controller 110 determines whether the number of times of retry performed thus far is equal to or less than a predetermined value in step S17 to determine whether to perform a retry. When it is determined to perform a retry (Y in step S17), the process moves to step S15. When it is determined not to perform a retry (N in step S17), the process proceeds to step S18.

### (S18)

When it is determined not to perform a retry (N in step S17), the controller 110 controls the second refractive power calculator 121B to obtains at least the spherical diopter power in the abovementioned manner. Step S18 is described in detail later. The process moves to step S5.

### (S19)

Having determined that no peak value is found in step S12 (N in step S12), the controller 110 changes the conditions of OCT measurement. For example, the controller 110 controls the spectrometer 83 to raise the detection sensitivity by a second change amount as a fourth example of change control in step S19. The second change amount may be a predetermined amount, or may be determined based on the maximum value of the peak value found by the search in step S11. The increase in the detection sensitivity facilitates the detection of interference light based on the return light from the fundus Ef of the subject's eye E.

In addition, as a fifth example of change control in step S19, the controller 110 may control the spectrometer 83 to increase the exposure time by second change time, or to increase the number of images, to be synthesized, based on light detected by the spectrometer 83 by a second change number. For example, the second change time and the second change number may be predetermined time and number, or may be determined based on the maximum value of the peak value found by the search in step S11. The increase in the exposure time or in the number of images to be synthesized facilitates the detection of interference light based on the return light from the fundus Ef of the subject's eye E.

Further, as a sixth example of change control in step S19, the controller 110 may control the OCT light source 81 to increase the light amount by a third change amount. For example, the third change amount may be a predetermined amount, or may be determined based on the maximum value of the peak value found by the search in step S11. The increase in the amount of light emitted from the light source increases the amount of light that reaches the fundus Ef of the subject's eye E. Thus, the amount of interference light based on the return light can be increased.

In step S19, at least one of the fourth to sixth examples of change control is performed. At least one of the fourth to sixth examples of change control to be performed may be selected based on the maximum value of the peak value found by the search in step S11. For example, one of the fourth to sixth examples of change control may be selected based on the maximum value of the peak value. Alternatively, priority order may be set for the fourth to sixth examples of change control in advance, and the control to be performed in step S15 may be determined according to the priority order.

### (S20)

In the same manner as in step S12, the controller 110 searches for a peak value, which is equal to or higher than a predetermined crest value, of the detection signal of the interference light LC generated by the measurement light LS returning from the fundus Ef of the subject's eye E, while moving the reference mirror 94B of the retinal reference mirror unit 94 along the optical axis.

### (S21)

When it is determined that a peak value is detected by the search of the peak value (Y in step S21), the process moves to step S13. When it is determined that a peak value is not detected by the search (N in step S21), the process proceeds to step S22.

### (S22)

Having determined that a peak value is not detected (N in step S21), the controller 110 determines whether the number of times of retry performed thus far is equal to or less than a predetermined value in step S22 to determine whether to perform a retry. When it is determined to perform a retry (Y in step S22), the process moves to step S19. When it is determined not to perform a retry (N in step S22), the process proceeds to step S23.

### (S23)

When it is determined not to perform a retry (N in step S22), the controller 110 performs a measurement error process. The measurement error process includes a process of displaying a notification of measurement error on the display unit 170. The measurement error process may include a process of displaying, on the display unit 170, a message indicating that a reliable value cannot be obtained by the present measurement. With this, the operation ends (END).

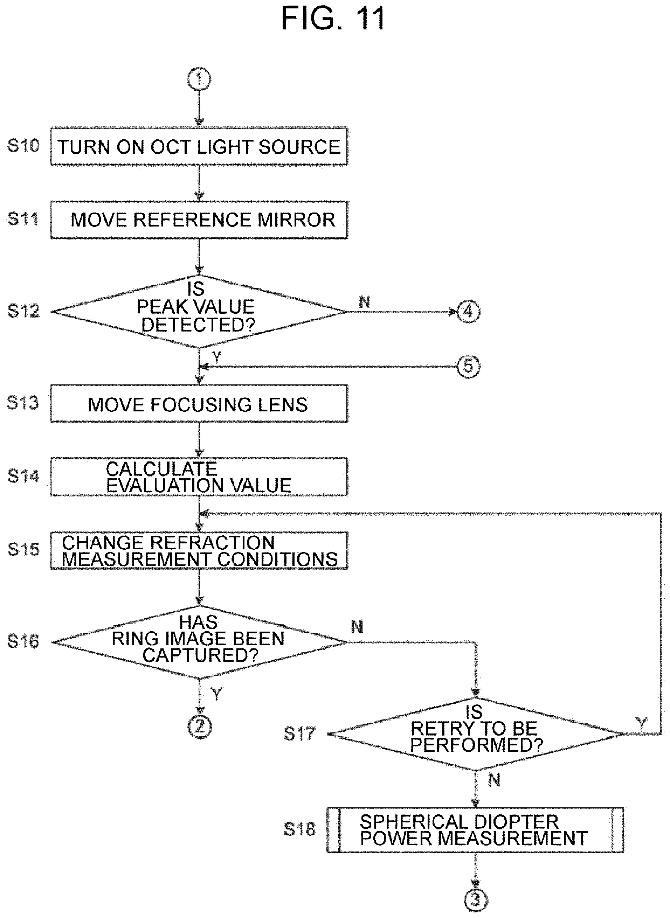

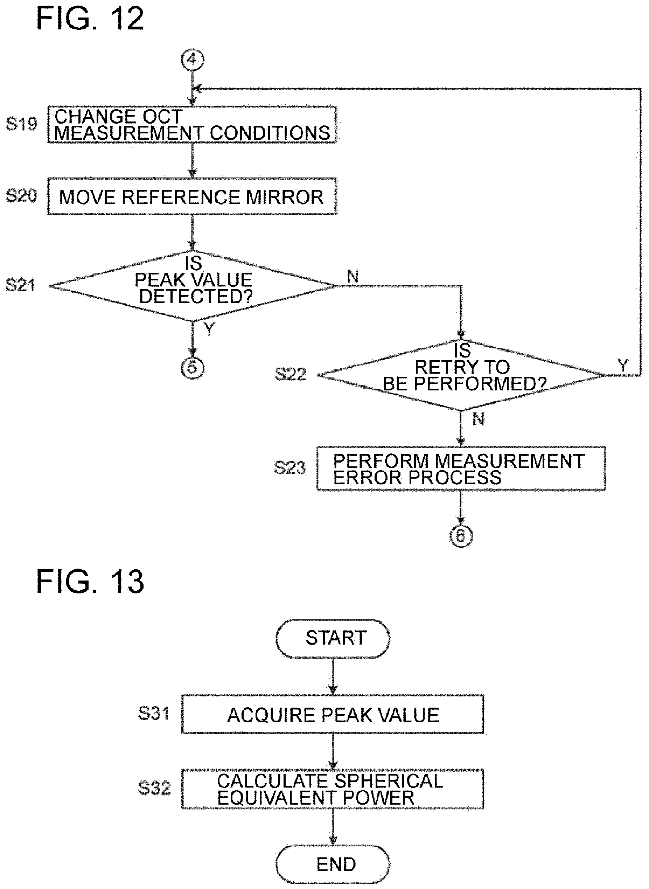

Fig 13 is a flowchart illustrating an example of the process of calculating the spherical diopter power in step S18 of Fig. 11.

### (S31)

When the process moves to step S18, the controller 110 acquires a peak value which is equal to or higher than a predetermined crest value from among peak values of detection signals of the interference light LC found by the search in step S11. Incidentally, if a peak value equal to or higher than the predetermined crest value has not been acquired, the highest value of the peak values may be used.

### (S32)

The controller 110 controls the second refractive power calculator 121B to obtain spherical equivalent power as the spherical diopter power. The second refractive power calculator 121B specifies the position of the focusing lens 85 where the peak value becomes the maximum in step S31, and obtains the spherical equivalent power based on the movement amount of the focusing lens 85 from the reference position corresponding to 0D. With this, the controller 110 ends the process of calculating the spherical diopter power (END).

Steps S10 to S22, S31, and S32 are an example of the "new measurement" of the embodiment. Steps S13 to S17 are an example of the "first measurement control" of the embodiment.

### <Effects>

Described below are the effects of the ophthalmologic apparatus 1000 according to the embodiment.

According to the embodiment, an ophthalmologic apparatus (e.g., the ophthalmologic apparatus 1000) includes a first measurement unit (e.g., the refractometry projection system 6, the refractometry light-receiving system 7, the first refractive power calculator 121A), a second measurement unit (e.g., the ocular axial length measurement system 8, the second refractive power calculator 121B), and a controller (e.g., the controller 110). The first measurement unit is configured to irradiate a subject's eye (e.g., the subject's eye E) with light from a first light source (e.g., the refractometry light source 61) and detect return light of the light returning from the subject's eye to measure a refractive power of the subject's eye. The second measurement unit is configured to split light from a second light source (e.g., the OCT light source 81) into reference light (e.g., the reference light LR) and measurement light (e.g., the measurement light LS), irradiate the subject's eye with the measurement light, and detect interference light (e.g., the interference light LC) between return light of the measurement light and the reference light to obtain at least spherical diopter power of the subject's eye. The controller is configured to control the second measurement unit to perform a new measurement based on a measurement result obtained by the first measurement unit.

With this configuration, when the refractive power of the subject's eye E cannot be measured by the first measurement unit, at least the spherical diopter power can be obtained by using the second measurement unit. Thus, even if the cataract has progressed, the power of IOL can be determined.

The first measurement unit may be configured to project a measurement pattern onto the subject's eye, and analyze the shape of a pattern image based on the return light from the subject's eye to measure the refractive power. The controller may be configured to control the second measurement unit to perform the new measurement based on a result of analysis of the shape of the pattern image.

With this configuration, it can be determined whether the first measurement unit can perform the measurement by analyzing the pattern image based on the return light of the measurement pattern. Thereby, when the refractive power of the subject's eye E cannot be measured by using the pattern image based on the return light of the measurement pattern, at least the spherical diopter power can be obtained. Thus, even if the cataract has progressed, the power of IOL can be determined.

The controller may be configured to perform first measurement control to analyze the shape of the pattern image obtained by performing re-measurement of the refractive power with changed measurement conditions of the first measurement unit before controlling the second measurement unit to perform the new measurement. In addition, the controller may be configured to, according to a result of analysis of the shape of the pattern image in the first measurement control, control the first measurement unit to perform the re-measurement or control the second measurement unit to perform the new measurement.

With this configuration, it is determined whether the first measurement unit can perform the measurement while changing the measurement conditions of the first measurement unit. Only when capable of performing the measurement, the first measurement unit performs the measurement again. Thus, a value as accurate as possible can be obtained by using the first measurement unit.

The controller may be configured to detect a peak position of the return light of the measurement light prior to controlling the second measurement unit to perform the new measurement. Further, the controller may be configured to perform the first measurement control if the peak position is detected. On the other hand, if the peak position is not detected, the controller may be configured to perform the first measurement control when the peak position of new return light is detected. Here, the new return light is obtained by measuring at least the spherical diopter power again by changing measurement conditions of the second measurement unit.

With this configuration, it is determined whether the second measurement unit can perform the measurement while the measurement conditions of the second measurement unit are being changed. Only when capable of performing the measurement, the second measurement unit performs the measurement again. Thus, a value as accurate as possible can be obtained by using the second measurement unit.

The first measurement unit may include a measurement optical system (e.g., the refractometry projection system 6, the refractometry light-receiving system 7), a first detector (e.g., the image sensor 74), and a first refractive power calculator (e.g., the first refractive power calculator 121A). The measurement optical system may be configured to irradiate the subject's eye with the light from the first light source, and guide return light from the subject's eye. The first detector may be configured to detect the return light guided by the measurement optical system. The first refractive power calculator may be configured to obtain the refractive power of the subject's eye based on a detection result of the return light obtained by the first detector. The controller may be configured to change at least one of the amount of the light from the first light source, the detection sensitivity of the first detector for detecting the return light, the exposure time of the return light in the first detector, and the number of images, to be synthesized, based on the return light detected by the first detector to change the measurement conditions of the first measurement unit.

With this configuration, the measurement conditions of the first measurement unit are changed by controlling the first light source and the first detector. Thus, when the cataract has progressed, the power of IOL can be determined with simple control.

The second measurement unit may include an interference optical system (e.g., the OCT unit 80), a second detector (e.g., the spectrometer 83), and a second refractive power calculator (e.g., the second refractive power calculator 121B). The interference optical system may be configured to split the light from the second light source into reference light and measurement light, irradiate the subject's eye with the measurement light, and generate interference light between the measurement light returning therefrom and the reference light. The second detector may be configured to detect the interference light. The second refractive power calculator may be configured to obtain at least the spherical diopter power based on a detection result of the interference light obtained by the second detector. The controller may be configured to change at least one of the amount of the light from the second light source, the detection sensitivity of the second detector for detecting the interference light, the exposure time of the interference light in the second detector, and the number of images, to be synthesized, based on the interference light detected by the second detector to change the measurement conditions of the second measurement unit.

With this configuration, the measurement conditions of the second measurement unit are changed by controlling the second light source and the second detector. Thus, when the cataract has progressed, the power of IOL can be determined with simple control.

The measurement pattern may be a ring-shaped pattern.

With this configuration, the first measurement unit measures the refractive power of the subject's eye by using a measurement pattern on a ring. Thus, even if the cataract has progressed, the power of IOL can be determined with a common refractometry method.

The second measurement unit may include an ocular axial length calculator (e.g., the ocular axial length calculator 122). The ocular axial length calculator may be configured to calculate the axial length of the subject's eye by detecting the interference light.

With this configuration, when the refractive power of the subject's eye E cannot be measured by the first measurement unit, at least the spherical diopter power is obtained by using the measurement result of the ocular axial length that is used to calculate the power of IOL. Thus, it is possible to downsize the apparatus for determining the power of IOL.

### <Modification>

In the above embodiment, an example is described in which the second refractive power calculator 121B obtains only the spherical diopter power (spherical equivalent power) by using the ocular axial length measurement system 8. However, the configuration of the ophthalmologic apparatus of the embodiment is not limited to this. According to a modification of the embodiment, the second refractive power calculator obtains the spherical diopter power, the astigmatic power, and the astigmatism axis angle with the use of the ocular axial length measurement system 8.

The ophthalmologic apparatus of the modification has basically the same configuration and operates in a similar manner to the ophthalmologic apparatus of the above embodiment. In the following, like reference numerals as those in the above embodiment are used to identify like parts, and the modification is described focusing on the differences from the embodiment.

In step S18 of Fig. 11, the ophthalmologic apparatus of the modification can determine the spherical diopter power, the astigmatic power, and the astigmatism axis angle with the ocular axial length measurement system 8 as described below.

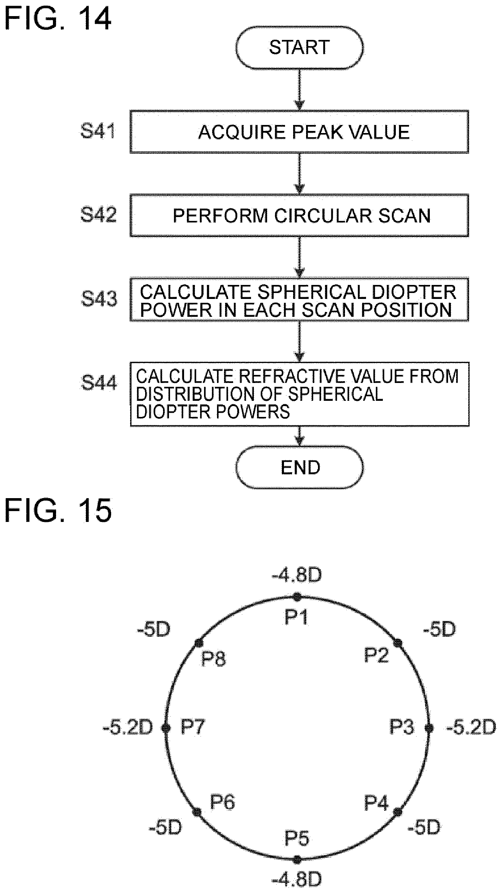

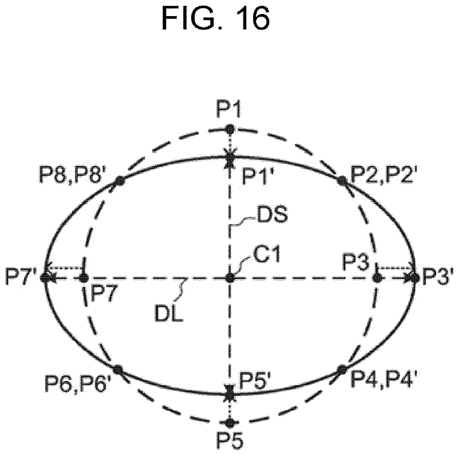

Fig. 14 is a flowchart illustrating an example of the process performed in step S18 of Fig. 11 according to this modification. Figs. 15 and 16 are diagrams for explaining the operation of steps S43 and S44 of Fig. 14.

### (S41)

In this modification, when the process moves to step S18, the controller 110 acquires a peak value which is equal to or higher than a predetermined crest value from among peak values of detection signals of the interference light LC found by the search in step S11. Incidentally, if such a peak value equal to or higher than the predetermined crest value has not been acquired, the highest value of the peak values may be used.

### (S42)

The controller 110 controls the XY scanner 87 to deflect the measurement light LS, thereby performing a circular scan over the pupil of the subject's eye E (e.g., on a circumference with a diameter of 3 mm) with the measurement light LS. For example, as illustrated in Fig. 15, scan positions P1 to P8 are scanned.

### (S43)

The second refractive power calculator 121B moves the focusing lens 85 to a position where the peak value becomes the maximum in each of the scan positions P1 to P8, and obtains the spherical diopter power from the position. In step S43, similarly to, for example, steps S31 and S32, the spherical diopter power (spherical equivalent power) is determined in each of the scan positions.

### (S44)

The second refractive power calculator 121B obtains the spherical diopter power, the astigmatic power, and the astigmatism axis angle (refractive values) from the distribution of spherical diopter powers each obtained in one of the scan positions in step S43.

In each of the scan positions, the aforementioned position of the focusing lens 85, that is, the spherical diopter power, is deviated by the astigmatic power. The second refractive power calculator 121B can analyze the spherical diopter power acquired in each of the scan positions P1 to P8 to obtain the spherical diopter power S, the astigmatic power C, and the astigmatism axis angle A of the subject's eye E. For example, the second refractive power calculator 121B calculates the average of spherical diopter powers each obtained in one of the scan positions in step S43, and associates the average value with the distance (radius of the circular scan) from the optical axis center position (barycentric position) C1 to each scan position. The second refractive power calculator 121B obtains the difference between the average value and the spherical diopter power for each of the scan positions. Using the center C1 as a reference, the second refractive power calculator 121B specifies new positions P1' to P8' taking into account the difference in each of the scan positions. The second refractive power calculator 121B performs an ellipse approximation on the new positions to specify an approximate ellipse AE (Fig. 16). The second refractive power calculator 121B obtains the spherical diopter power S, the astigmatic power C, and the astigmatism axis angle A according to a known equation using the length of the long diameter DL and that of the short diameter DS of the approximate ellipse AE.

With this, the controller 110 ends the process of step S18 of Fig. 11 according to this modification (END).

With this configuration, even if the refractive power of the subject's eye E cannot be measured by a refractometry, the spherical diopter power S, the astigmatic power C, and the astigmatism axis angle A can be obtained by using the ocular axial length measurement system. Thus, even if the cataract has progressed, the power of IOL can be obtained with higher accuracy as compared to the conventional process.

### (Other Modifications)

The embodiments described above are mere examples for embodying or carrying out the present invention, and therefore susceptible to several modifications and variations (omission, substitution, addition, etc.), all coming within the scope of the invention.

In the above embodiment and the modification thereof, an example is described in which the ocular axial length is obtained by using the ocular axial length measurement system. Similarly, the corneal thickness, the anterior chamber depth, the lens thickness, or the like can also be obtained with the use of the ocular axial length measurement system.

The optical elements and their arrangement are not limited to those described in the embodiment and the modification thereof. For example, the half mirror of the embodiment and the modification may be replaced with any kind of beam splitters.

The above embodiment and the modification thereof can be applied to an apparatus having any function that can be used in the ophthalmic field, such as intraocular pressure measurement function, fundus photography function, anterior eye segment photography function, optical coherence tomography (OCT) function, ultrasonic test function, and the like. Incidentally, the intraocular pressure measurement function is realized by a tonometer or the like. The fundus photography function is realized by a fundus camera, a scanning ophthalmoscope (SLO) or the like. The anterior eye segment photography function is realized by a slit lamp or the like. The OCT function is realized by any type of optical coherence tomography or the like. The ultrasonic test function is realized by an ultrasonic diagnostic apparatus or the like. The embodiment and the modification can also be applied to an apparatus (multifunctional ophthalmologic apparatus) having two or more of such functions.

Further, in a system to which the above embodiment and the modification thereof can be applied, the embodiment and the modification can be alternatively applied by switching the operation modes.
The invention is further defined by the following items:
1. An ophthalmologic apparatus comprising:
   a first measurement unit configured to irradiate a subject's eye with light from a first light source and detect return light thereof from the subject's eye to measure a refractive power of the subject's eye;
   a second measurement unit configured to split light from a second light source into reference light and measurement light, irradiate the subject's eye with the measurement light, and detect interference light between return light of the measurement light from the subject's eye and the reference light to obtain at least spherical diopter power of the subject's eye; and
   a controller configured to control the second measurement unit to perform a new measurement based on a measurement result obtained by the first measurement unit.
2. The ophthalmologic apparatus of item 1, wherein
   the first measurement unit is configured to project a measurement pattern onto the subject's eye, and analyze a shape of a pattern image based on return light thereof from the subject's eye to measure the refractive power, and
   the controller is configured to control the second measurement unit to perform the new measurement based on a result of analysis of the shape of the pattern image.
3. The ophthalmologic apparatus of item 2, wherein the controller is configured to
   perform first measurement control to analyze the shape of the pattern image obtained by changing measurement conditions of the first measurement unit and performing re-measurement of the refractive power before controlling the second measurement unit to perform the new measurement, and
   according to the result of the analysis of the shape of the pattern image in the first measurement control, control the first measurement unit to perform the re-measurement or control the second measurement unit to perform the new measurement.
4. The ophthalmologic apparatus of item 3, wherein the controller is configured to
   detect a peak position of the return light of the measurement light before controlling the second measurement unit to perform the new measurement,
   perform the first measurement control when the peak position is detected, and
   when the peak position is not detected and when a peak position of new return light is detected, the new return light being obtained by changing the measurement conditions of the second measurement unit and controlling the second measurement unit to perform the re-measurement of at least the spherical diopter power, perform the first measurement control.
5. The ophthalmologic apparatus of item 3 or 4, wherein
   the first measurement unit includes:
   a measurement optical system configured to irradiate the subject's eye with the light from the first light source, and guide the return light thereof;
   a first detector configured to detect the return light guided by the measurement optical system; and
   a first refractive power calculator configured to obtain the refractive power of the subject's eye based on a detection result of the return light obtained by the first detector, and
   the controller is configured to change at least one of amount of the light from the first light source, detection sensitivity of the first detector for detecting the return light, exposure time of the return light in the first detector, and number of images, to be synthesized, based on the return light detected by the first detector to change the measurement conditions of the first measurement unit.
6. The ophthalmologic apparatus of any one of items 3 to 5, wherein
   the second measurement unit includes:
   an interference optical system configured to split the light from the second light source into the reference light and the measurement light, irradiate the subject's eye with the measurement light, and generate the interference light between the return light of the measurement light and the reference light;
   a second detector configured to detect the interference light; and
   a second refractive power calculator configured to obtain at least the spherical diopter power based on a detection result of the interference light obtained by the second detector, and
   the controller is configured to change at least one of amount of the light from the second light source, detection sensitivity of the second detector for detecting the interference light, exposure time of the interference light in the second detector, and number of images, to be synthesized, based on the interference light detected by the second detector to change the measurement conditions of the second measurement unit.
7. The ophthalmologic apparatus of any one of items 2 to 6, wherein the measurement pattern is a ring-shaped pattern.
8. The ophthalmologic apparatus of any one of items 1 to 7, wherein the second measurement unit includes an ocular axial length calculator configured to calculate an axial length of the subject's eye by detecting the interference light.

## Claims

1. Method for testing a subject's eye with an optical apparatus having an optical system, the optical system comprising an ocular axial length measurement system (8), a topographic measurement unit (3), a refractometry measurement unit (6), and a processor, wherein a central axial length and a peripheral axial length of subject's eye is measured with the ocular axial length measurement system (8), wherein the topographic measurement unit (3) measures the shape of the cornea (K) of the eye, wherein the refractometry measurement unit (6) measures a refractive power of the subject's eye, wherein the processor (9) processes measurement data of the ocular axial length measurement system (8), the topographic measurement unit (3), and the refractometry measurement unit (6), wherein the processor (9) outputs the measurement data.

2. Method according to claim 1, wherein a central fixation mark is displayed for the eye in the infinite by the fixation system (4), wherein the central fixation is focussed by the eye and a fixation of the eye is performed relative to the optical system.

3. Method according to any one of the preceding claims, wherein the measurements with the ocular axial length measurement system (8), the topographic measurement unit (3), and the refractometry measurement unit (6) are concurrently performed at the eye.

4. Method according to any one of the preceding claims, wherein the processor (9) performs a comparison of the central axial length measurement data and the peripheral axial length measurement data and determines a degree of refraction by said comparison.

5. Ophthalmologic apparatus (1000) including an optical system for testing a subject's eye, comprising:
an ocular axial length measurement system (8),
a topographic measurement unit (3),
a refractometry measurement unit (6), and
a processor (9),
wherein the ocular axial length measurement apparatus (8) is configured to measure a central axial length and a peripheral axial length of subject's eye,
wherein the topographic measurement unit (3) is configured to measure the shape of the cornea (K) of the eye,
wherein the refractometry measurement unit (6) is configured to measure a refractive power of the subject's eye,
wherein the processor (9) is configured to process measurement data of the ocular axial length measurement system (8), the topographic measurement unit (3), and the refractometry measurement unit (6),
wherein the optical system further comprises a fixation system (4),
wherein the eye is selectively fixable by the fixation system (4) relative to the optical system for measurement of the central axial length or the peripheral axial length.

6. Ophthalmologic apparatus (1000) of claim 5, wherein the ocular axial length measurement system (8), the topographic measurement unit (3), and the refractometry measurement unit (6) are integrated in the apparatus.

7. Ophthalmologic apparatus (1000) of claims 5 or 6, wherein the fixation system (4) comprises a central fixation mark that is displayable for the eye in the infinite and focusable by the eye, wherein the central fixation mark is arranged such that, when the central fixation is focussed by the eye, the viewing axis of the eye is flush with the optical axis of ocular axial length measurement system (8).

8. Ophthalmologic apparatus (1000) of claim 5 bis 6, wherein the ocular axial length measurement system (8), the topographic measurement unit (3), and the refractometry measurement unit (6) have a common measurement axis that can be made coincident with the optical axis of the eye.

9. Ophthalmologic apparatus (1000) of claim 5 bis 8, wherein the ocular axial length measurement system (8) is an ultrasonic measurement system or an interferometric measurement system.

10. Ophthalmologic apparatus (1000) of claim 9, wherein the ocular axial length measurement system (8) is an interferometer for optical coherence tomography, OCT.

11. Ophthalmologic apparatus (1000) of claim 5 bis 10, wherein the topographic measurement unit (3) is a keratometry measurement unit (3).

12. Ophthalmologic apparatus (1000) of claim 5 bis 11, wherein the refractometry measurement unit (6) is an auto-refractometer.
